# EUROPEAN PATENT SPECIFICATION

(11) **EP 2 586 482 B1**
(45) Date of publication and mention of the grant of the patent: **10.02.2016**
(21) Application number: 11186276.9
(22) Date of filing: 24.10.2011
(51) Int. Cl.: A61M 16/22, A62B 19/00

(54) **Housing assembly for substance removing an undesired respiratory gas component of a respiratory gas flow and arrangement for ventilating lungs of a subject**
Gehäuseanordnung für eine Substanz zur Entfernung eines unerwünschten Atemgasbestandteils aus einem Atemgasstrom und Anordnung zur Beatmung der Lungen eines Patienten
Ensemble de boîtier avec une substance pour éliminer d'un composant indésirable du gaz respiratoire d'un flux de gaz respiratoire et agencement pour ventiler les poumons d'un sujet

(43) Date of publication of application: 01.05.2013
(73) Proprietor: General Electric Company, Schenectady, NY 12345 (US)
(72) Inventor: Heinonen, Erkki, 00750 Helsinki (FI); Räty, Marko, 04500 Kellokoski (FI); Alanen, Mikael, 02650 Espoo (FI); Pekkarinen, Tomi, 02970 Espoo (FI)
(74) Representative: Illingworth-Law, William Illingworth

(56) References cited:
- EP-A2- 1 230 943
- CA-A- 668 081

## Description

### BACKROUND OF THE INVENTION

The disclosure relates generally to a housing assembly for a substance removing an undesired respiratory gas component of a respiratory gas flow. The housing assembly includes a body having a space for receiving the substance and having a first end and a second end, which is opposite the first end, allowing the respiration gas flow between these ends, a first wall surrounding at least part of the space, and a first opening in the flow communication with the first end and with outside the housing. The housing assembly includes a channel in flow communication with the second end and which channel includes a passage surrounded at least partly by a second wall for guiding the gas flow, and a second opening in flow communication with the passage. The disclosure also relates to an arrangement for ventilating lungs of a subject.

Anesthesia machines are optimized for delivering anesthesia to a patient using volatile anesthetic agent liquids. In such systems, the anesthetic agent is vaporized and mixed into the breathing gas stream in a controlled manner to provide a gas mixture for anesthetizing the patient for a surgical operation. The most common volatile anesthetic agents are halogenated hydrocarbon chains, such as halothane, enflurane, isoflurane, sevoflurane and desflurane. Additionally, nitrous oxide (N₂O) can be counted in this group of volatile anesthetic agents, although the high vapor pressure of nitrous oxide causes nitrous oxide to vaporize spontaneously in the high pressure gas cylinder, wherefrom it can be directly mixed as gas with oxygen. The anesthetizing potency of nitrous oxide alone is seldom enough to anesthetize a patient and therefore another volatile agent is used to support that.

Since the volatile anesthetic agents are expensive, they are effective greenhouse gases and further harmful to the atmospheric ozone layer, anesthesia machines have been developed to minimize the consumption of the gases. To keep patients anesthetized, a defined brain partial pressure for the anesthetic agent is required. This partial pressure is maintained by keeping the anesthetic agent partial pressure in the lungs adequate. During a steady state, the lung and body partial pressures are equal, and no net exchange of the anesthetic agent occurs between the blood and the lungs. However, to provide oxygen and eliminate carbon dioxide, continuous lung ventilation is required.

Anesthesia machines are designed to provide oxygen to the patient and eliminate carbon dioxide (CO2), while preserving the anesthetic gases. To meet these goals a re-breathing circuit is used. In this patient exhaled gas is reintroduced for inhalation. Before re-inhalation carbon dioxide must be removed from the gas, which is done with a carbon dioxide absorber. Before inhalation, the gas is supplied with additional oxygen and anesthetic agents based upon the patient demand. In this arrangement, the additional gas flow added to the re-breathing circuit can be less than 0.5 L/min although the patient ventilation may be 5-10 L/min. Such ventilation of the lung is carried out using a ventilator pushing inhalation gas with overpressure to patient lungs and then allowing that to flow out passively from the pressurized lungs to the breathing circuit.

Ventilation carries the breathing circuit oxygen to lungs for uptake to be burned in body metabolism. The outcome of this is CO2 that blood circulation transports to lungs wherefrom it becomes carried out with exhalation gas. Before re-inhalation the gas is guided through absorber for CO2 removal. Effective CO2 removal requires close contact with the breathing gas and the removing substance. To provide large contact area, the removing substance is therefore a surface of porous structure of granules that fill the cartridge. The form of this granular structure is guided by the flow resistance, the limitation of which is one key design goals of the breathing circuit. In absorber optimized for minimal resistance the gas flow path through the stacked granules is short and the flow distributes to wide area. In such structure the gas flows slowly because of large surface area providing time for reaction between the gas and absorbent granules.

The absorbers tend to have empty space above the granules. If the gas inlet and outlet to the absorber would be aligned to allow horizontal flow penetration through the cartridge, the flow would favor this empty space and the CO2 would leak through without getting absorbed. Therefore the gas flow must always penetrate through the absorbent on vertical direction.

Absorber canisters have two gas connections: One inlet for the gas flow carrying carbon-dioxide and one outlet. Between inlet and outlet the canister has a gas pathway. The absorber granules form part of this pathway during which the carbon dioxide is removed from the gas.

Loose absorbent granules are difficult to handle. Therefore these are increasingly pre-packed in disposable housing including the absorbent. Being disposable the housing needs to be economical to manufacture and package for transport from the manufacturing plant to the operating room. The economical manufacturing involves robust design for streamlined housing production and minimizing manufacturing scrap costs. The economical packaging involves limitation of space-requiring extensions to functional minimum.

The pre-packed canisters may weight over 1 kg thus the design must take into consideration the handling during assembly and disassembly to the breathing circuit. This assembly must provide gas-tight connection between the cartridge and the rest of the breathing system taking into consideration of all manufacturing tolerances within the breathing system and the disposable absorber.

The absorber canister interfaces with breathing system through valves that automatically guide the gas flow through the absorber when the absorber is installed on place and re-directs to absorber bypass channel when the canister is removed. Once installed on place the canister must seal to the receptacles on the breathing circuit to allow tight connection. This sealing requirement sets tight tolerances for the joint to mate exactly the breathing circuit and the canister. Low cost requirement of disposable canister however does not allow tight manufacturing tolerances.

CA 668081 discloses an absorber removing carbon dioxide from respiratory gases of patients including an upper and lower housing to secure a vertically arranged series of canisters. The absorber has an inlet to guide exhalation gas downwardly through the canisters to remove carbon dioxide, a flexible corrugated tubing to guide this gas to an outlet and through this outlet back for inhalation. The corrugations of the tubing expand when the lower housing is lowered to remove the upper canister and to replace with a fresh one.

### BRIEF DESCRIPTION OF THE INVENTION

The above-mentioned shortcomings, disadvantages and problems are addressed herein which will be understood by reading and understanding the following specification.

The present invention is defined in the accompanying claims.

In an embodiment, a housing assembly for a substance removing an undesired respiratory gas component of a respiratory gas flow includes a body having a space for receiving the substance and having a first end and a second end, which is opposite the first end, allowing the respiration gas flow between these ends, a first wall surrounding at least part of the space, and a first opening in the flow communication with the first end and with outside the housing. The housing assembly also includes a channel in flow communication with the second end and which channel includes a passage surrounded at least partly by a second wall for guiding the gas flow, and a second opening in the flow communication with the passage and with outside the housing. At least part of the channel is adapted to be apart from the body enabling a variation in respect of mutual location of the first opening and the second opening.

In another embodiment, an arrangement for ventilating lungs of a subject includes a ventilator for supplying a breathing gas for an inspiration and for receiving a breathing gas for an expiration and a gas mixer for supplying a fresh gas for the subject breathing. The arrangement also includes a breathing circuit for connecting lungs of the subject, the ventilator and the gas mixer, the breathing circuit including an inspiration limb providing an inspiration gas including the fresh gas for the subject breathing, an expiration limb to discharge an expiration gas, and a housing assembly for a substance removing an undesired respiratory gas component of a respiratory gas flow. The housing assembly includes a body having a space for receiving the substance and having a first end and a second end, which is opposite the first end, allowing the respiration gas flow between these ends, a first wall surrounding at least part of the space, and a first opening in the flow communication with the first end and with outside the housing. The housing assembly also includes a channel in flow communication with the second end and which channel includes a passage surrounded at least partly by a second wall for guiding the gas flow, and a second opening in the flow communication with the passage and with outside the housing. At least part of the channel is adapted to be apart from the body enabling a variation in respect of mutual location of the first opening and the second opening.

In yet another embodiment, a housing assembly for a substance removing an undesired respiratory gas component of a respiratory gas flow includes a body having a space for receiving the substance and having a first end and a second end, which is opposite the first end, allowing the respiration gas flow between these ends, a first wall surrounding at least part of the space, and a first opening adapted to be in the flow communication with the first end and with outside the housing. The housing assembly also includes a channel in flow communication with the second end and which channel includes a passage surrounded at least partly by a second wall for guiding the gas flow, and a second opening in the flow communication with the passage and with outside the housing. At least part of the channel is adapted to be apart from the body enabling a variation in respect of mutual location of the first opening and the second opening and which channel is adapted to act as handle for gripping the housing assembly by a hand.

Various other features, objects, and advantages of the invention will be made apparent to those skilled in art from the accompanying drawings and detailed description thereof.

### BRIEF DESCRIPTION OF THE DRAWINGS

Figure 1 illustrates an operational diagram of an arrangement for ventilating lungs of a subject;
Figure 2 is a schematic cross-sectional view of a housing assembly for a solid fluidal substance removing an undesired respiratory gas component of a respiratory gas flow in accordance with an embodiment;
Figure 3 is a detail embodiment of the housing assembly of Figure 2 to support the structure; and
Figure 4 is a side-view of the housing assembly of Figure 2.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

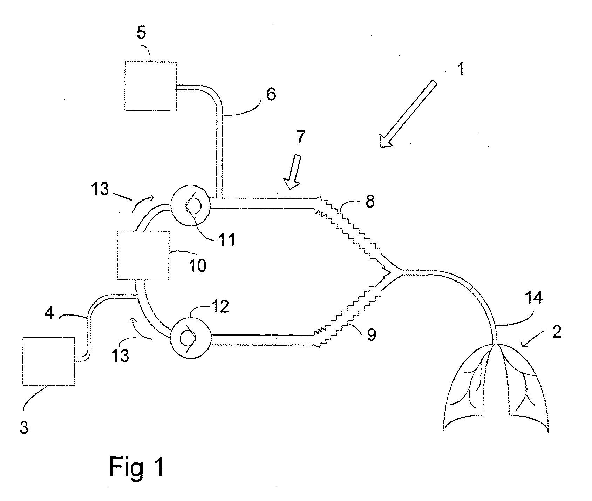

In Fig. 1 an arrangement 1 for ventilating lungs 2 of a subject is disclosed. The arrangement comprises a ventilator 3 supplying in this specific embodiment along a ventilator connection 4 breathing gas to the lungs for an inspiration and receiving breathing gas for expiration. The ventilator may be whichever well-known type e.g. drive gas based pneumatic flow-valve or mechanical piston driven. Also the arrangement comprises a gas mixer 5 supplying a fresh gas in this specific embodiment along a fresh gas tube 6 for the subject breathing, a breathing circuit 7 connecting lungs of the subject 2, the ventilator 3 and the gas mixer 5. The gas mixer may comprise an anesthetic agent supply (not shown in the figure) such as an anesthetic agent vaporizer providing anesthetic agent for the subject breathing.

The breathing circuit 7, which may be a re-breathing circuit, comprises an inspiration limb 8 providing an inspiration gas including the fresh gas for the subject breathing and an expiration limb 9 discharging an expiration gas. The ventilator is controlling the breathing circuit pressure through the ventilator tube 4. Also the breathing circuit comprises a housing assembly 10 for substance, which may be solid fluidal material, such as granules for removing an undesired respiratory gas component of a respiratory gas flow. Typical substance used in anesthesia is a carbon dioxide absorbing material, which may be soda-lime, a mixture of calcium hydroxide, sodium hydroxide, potassium hydroxide and water or any other substance that can extract CO2 from gas mixture e.g. molecular sieve or amines. The material may chemically react with carbon dioxide or otherwise remove it from the breathing gas. The housing assembly 10 is detachable from the breathing circuit.

Typically the breathing circuit 7 also comprises directional valves 11 and 12 guiding the gas flow in the circuit on direction indicated by arrows 13. For inhalation the ventilator 3 increases the breathing circuit pressure by adding the gas flow from ventilator connection 4. Directional valves 11 and 12 guide the gas flow through the housing assembly 10, including the substance removing in this embodiment carbon dioxide from the breathing gas, to the inspiration limb 8 and further along a subject limb 14 to the subject's lungs 2. For expiration the ventilator 3 releases gases from the breathing circuit through the ventilator connection 4. For this purpose the ventilator 3 may e.g. operate an expiration valve (not shown in Figure). This will allow the gas flow from distended subject's lungs through the subject limb 14 to the expiration limb 9 and further through the directional valve 12 to the ventilator tube 4. The directional valve 11 prevents the gas flow from the subject's lungs to enter the inspiration limb 8 hereby maintaining the inspiration limb free from CO2. Instead, the exhaled gas is rich of CO2 that needs to be removed before being re-circulated for the inspiration, which is done in the housing assembly 10 including the substance removing carbon dioxide.

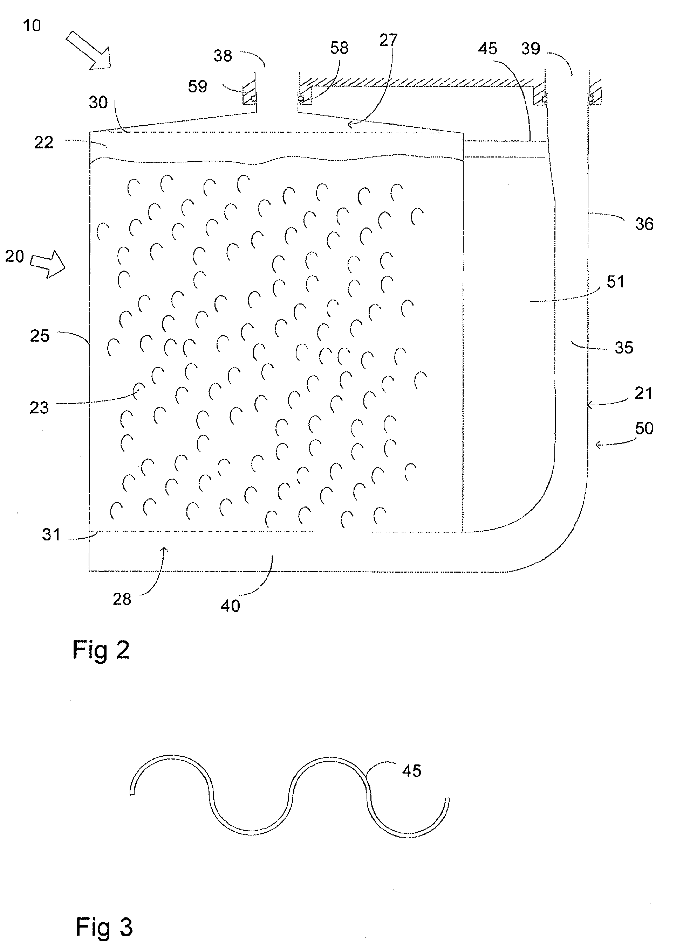

The housing assembly 10 of Fig 2 comprises body 20 and a channel 21 for the gas flow. The body comprises a space 22 for receiving a substance 23 removing an undesired respiratory gas component of a respiratory gas flow and a first wall 25 surrounding at least partly the space 22. Also the body 20 comprises two opposite ends, which are a first end 27, such as an upper end, and a second end 28, such as a lower end, allowing the respiratory gas flow between these ends and through these ends and which ends leave therebetween the space 22. There are in both ends shields, which are the first shield 30, such as an upper shield, and the second shield 31, such as a lower shield.. The space 22 between the shields is filled at least partially with the substance 23 such as the fluidic granular CO2 absorbing substance. These shields are structures to prevent the absorbent substance to escape from the body 20 but allowing gas flow through. The shield maybe a rigid structure alone or a rigid structure that supports separate filter structure (not shown). The filter structure can be any form of structure having porosity for the gas flow, e.g. a foam or woven material. The first and second shield structures may be the same or different.

The channel 21, such as an open gas channel, comprises a passage 35 for the gas flow surrounded at least partly by a second wall 36 and which channel is in flow communication with the space 22 of the body 20. This channel is operationally connected to the second end 28 of the body 20, which is in the lower part of the body in Fig. 2. The channel and the body are side by side, whereupon the gas is flowing, as the case is in Figure 2 embodiment, first downwards along one of the space 22 and the passage 35 and then the flow is turned upwards flowing along the remaining one of the space and the passage 35. Or if the housing assembly 10 is upside down the gas is flowing first up along one of the space 22 and the passage 35 and then the flow is turned downwards flowing along the remaining one of the space and the passage 35. So the gas is flowing along the space 22 and the passage 35 in opposite vertical directions. As evident hereinbefore the gas flow in the body and the channel can be in either direction depending on the design meaning that the gas flow can be from the passage 35 towards the space 22 or from the space towards the passage.

The body also comprises a first opening 38 being in flow communication, advantageously in direct flow communication, with the first end and with outside the housing assembly and the channel 21 comprises a second opening 39 being in flow communication, advantageously in direct flow communication, with the passage and outside the housing assembly. Thus these openings provide a gas flow communication between the anesthesia breathing circuit 7 and both the space 22 and the passage 35. In communication with the breathing circuit one of these openings acts as a gas flow inlet and the other as a gas flow outlet. The gas flow communication between the first opening and the second opening comprise of the open gas flow channel 21, the body 20, the first shield 30 in the first end 27 and the second shield 31 in the second end 28.

Between the first shield 30 and the first opening 38 there may be an open volume in the first end 27 allowing the gas flow throughout the whole space 22 of the body horizontal cross section to communicate with the gas flow at the first opening 38 aiming for even flow velocity profile across this horizontal cross section. Similarly, between the second shield 31 and the passage 35 there may be a chamber 40 in the second end 28 that allows gas flow communication between the space 22 and this passage and opening to whole horizontal cross sectional area of the space 22 for the gas flow to ensure a constant flow velocity profile throughout the horizontal cross sectional area of the space 22.

At least part of the channel 21 is apart from the body 20 enabling to vary mutual location of the first opening 38 and the second opening 39. The horizontal distance between the first opening and the second opening, which openings are parallel, is changing at least in some degree when these openings are opening into the same direction, which is typically either upwards or downwards when connected to the breathing circuit 7. The distance may vary at least ± 0,1mm, more specifically at least ± 0,3 mm or even more specifically at least ± 0,5 mm. The variation in respect of the mutual location of the first opening and the second opening or the body 20 and the channel 21 can be made for example by providing at least partly an elastic element 45, such as an intermediate wall, between the channel 21 and the body 20, which are parallel, connecting them together, but allowing their mutual distance to change. In that case at least some part of the first wall 25 connects to the second wall 36 by means of the elastic element 45. The elastic element can have a form of spring that can be injection molded readily to the body 20. Also it is possible that at least part of one of the channel and the body comprise the elastic element enabling bending resulting in variation between horizontal mutual location of the first opening and the second opening when the housing assembly is in an operation position. However, it might be reasonable that at least part of the channel 21, such as a joint between the body and the channel, is made of an elastic material forming the elastic element enabling to bend the channel varying the mutual location of the first opening 38 and the second opening 39. In this specific case the mutual location of the body and the channel is varying. Meanwhile allowing mutual horizontal movement, the joint preferably maintains the vertical positioning of the openings 38 and 39 unchanged when in operation position.

The channel 21 also acts as a handle 50 for convenient gripping the housing assembly 10 by hand in which case the second opening may be at the end of the handle. For this purpose between the body 20 and the channel 21 remains a finger space 51 to provide a firm touch of the housing assembly. This finger space provides advantageously open access for finger- and palm touch around handle formed by the channel 21. To give mechanical rigidity for the housing assembly with the handle the finger space may be supported with the elastic element 45 connecting the channel 21 and the body 20. This connection may close the finger space advantageously partially as indicated on the Fig 2 or totally. Advantageously the handle 50 is formed oval from the touch area. This provides additional finger space between the handle and the body and gives more rigid touch. Such combination also simplifies the housing assembly and minimizes its size. The oval dimensions are advantageously (40-55)mm x (12-20)mm and more specifically (45-50)mm x (12-15)mm. The finger space 51 between the first wall 25 of the body 20 and the second wall 36 of the channel 21 is advantageously at least 10 mm, more specifically 20 mm or even more specifically 30 mm.

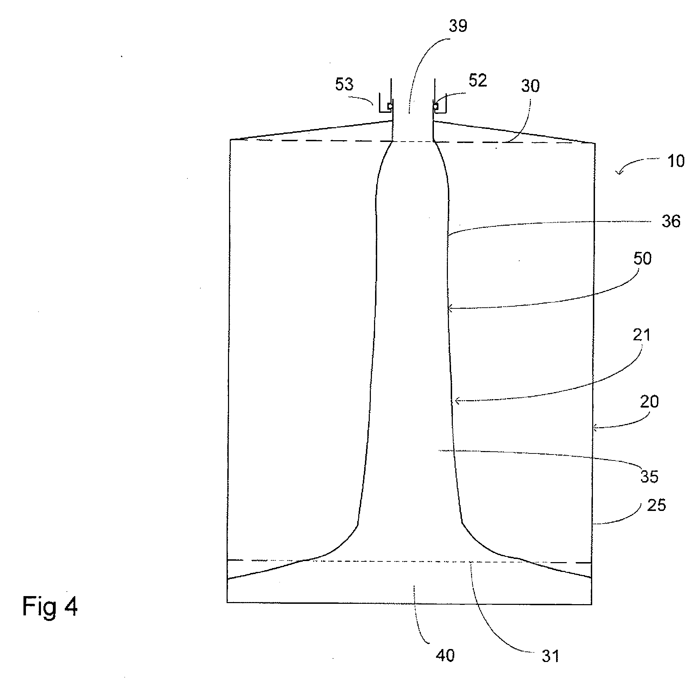

Fig 4 gives a side-view of the housing assembly 10 to show the handle-side. This presents advantageous broadening of the handle 50 at the touch area making the profile flat. This improves the touch compared to cylindrical cross-sectional profile and gives more room for fingers between the body 20 and the channel 21, which is illustrated on Fig 2. At the same time the open flow channel cross-sectional area remains to avoid increased gas flow resistance. Towards the top of the open flow channel the flattened profile is advantageously reformed to circular one to ensure leak-tight sealing to the breathing circuit. The seals 52 of the connectors may be part of the breathing circuit receptacle 53 or advantageously they are part of the housing assembly 10. In the latter the seals 52 become changed every time the housing assembly is changed thus ensuring the leak-tight connection.

To allow required tolerance to adopt manufacturing variability between different breathing circuits and housing assemblies and to allow leak-tight connection between the breathing circuit and the first and second openings 38, 39 of the housing assembly 10, the second opening 39 at the top of open flow channel 21 is made flexibly moveable in relation to the first opening 38 at the top of the body 20. Fig 3 presents as top view an example of the elastic element 45 such as an intermediate wall that at the same time provides flexibility between the mutual distance of the body 20 and handle 50 for firm touch of the housing assembly. In this Fig 3 the elastic element 45 is made to comprise wave-formed structures that allow at the same time firm mounting and variability in mutual distance between the first opening 38 and the second opening 39.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A housing assembly for a substance (23) removing an undesired respiratory gas component of a respiratory gas flow comprising:
a body (20) having a space (22) for receiving said substance and having a first end (27) and a second end (28), which is opposite said first end,
allowing the respiration gas flow between these ends, a first wall (25) surrounding at least part of said space, and a first opening (38) in the flow communication with said first end and with outside said housing;
a channel (21) in flow communication with said second end and which channel comprises a passage (35) surrounded at least partly by a second wall (36) for guiding the gas flow, and a second opening (39) in the flow communication with said passage and with outside said housing;
**characterized in that** said at least part of said channel is adapted to be apart from said body to enable a variation in respect of mutual location of said first opening and said second opening and **in that** the channel is adapted to act as a handle (50) for the body.

2. The housing assembly according to claim 1, **characterized in that** said variation in respect of mutual location of said first opening and said second opening includes a change in a distance between said first opening and said second opening, which distance may vary at least ± 0,1 mm, more specifically at least ± 0,3 mm or even more specifically at least ± 0,5 mm.

3. The housing assembly according to claim 1, **characterized in that** from one of said first opening and second opening the gas is adapted to flow towards said space and the remaining one of said first opening and said second opening is for removing the gas from said space.

4. The housing assembly according to claim 1, **characterized in that** said first opening and second opening are opening into same direction.

5. The housing assembly according to claim 1, **characterized in that** said housing also comprises an elastic element (45) between said first opening and said second opening.

6. The housing assembly according to claim 5, **characterized in that** said elastic element is adapted to connect said second wall to said first wall.

7. The housing assembly according to claim 5 or 6, **characterized in that** said elastic element is an intermediate wall between said second wall and said first wall.

8. The housing assembly according to claim 1, **characterized in that** at least part of one of said channel and said body comprise said elastic element enabling bending resulting in variation between mutual location of said first opening and said second opening.

9. The housing assembly according to claim 1, **characterized in that** said first opening and second opening are parallel.

10. The housing assembly according to claim 1, **characterized in that** a finger space (51) is provided between said channel and said body.

11. An arrangement for ventilating lungs of a subject comprising:
a ventilator (3) for supplying a breathing gas for an inspiration and for receiving a breathing gas for an expiration;
a gas mixer (5) for supplying a fresh gas for the subject breathing; and
a breathing circuit (7) for connecting lungs (2) of the subject, said ventilator and said gas mixer, said breathing circuit comprising an inspiration limb (8) providing an inspiration gas including the fresh gas for the subject breathing; an expiration limb (9) to discharge an expiration gas; and a housing assembly (10) according to claim 1.

12. The arrangement according to claim 11, **characterized in that** said first opening and said second opening, which are adapted to be in flow communication with outside said housing, are adapted to be in flow communication with said breathing circuit and that said first opening and second opening are opening into same direction.

13. The housing assembly according to claim 11, **characterized in that** said variation in respect of mutual location of said first opening and said second opening includes a change in a horizontal distance between said first opening and said second opening.

## Patentansprüche

1. Gehäuseanordnung für eine Substanz (23), die einen unerwünschten Atemgasbestandteil eines Atemgasflusses entfernt, umfassend:
einen Körper (20), der einen Raum (22) zum Empfangen der Substanz aufweist und ein erstes Ende (27) und ein zweites Ende (28) aufweist, das gegenüber dem ersten Ende liegt, das den Atemgasfluss zwischen diesen Enden ermöglicht, wobei eine erste Wand (25) mindestens einen Teil des Raumes umgibt, und eine erste Öffnung (38) in Strömungsverbindung mit dem ersten Ende und dem Äußeren des Gehäuses aufweist;
einen Kanal (21) in Strömungsverbindung mit dem zweiten Ende und welch Kanal einen Durchlass (35), der mindestens teilweise von einer zweiten Wand (36) zum Lenken des Gasflusses umgeben ist, und eine zweite Öffnung (39) in Strömungsverbindung mit dem Durchlass und dem Äußeren des Gehäuses umfasst;
**dadurch gekennzeichnet, dass** mindestens ein Teil des Kanals angepasst ist, um vom Körper entfernt zu sein, um einen Unterschied in Bezug auf gegenseitige Lage der ersten Öffnung und der zweiten Öffnung zu ermöglichen und dadurch, dass der Kanal angepasst ist, um als Griff (50) für den Körper zu fungieren.

2. Gehäuseanordnung nach Anspruch 1, **dadurch gekennzeichnet, dass** der Unterschied in Bezug auf gegenseitige Lage der ersten Öffnung und der zweiten Öffnung eine Änderung einer Entfernung zwischen der ersten Öffnung und der zweiten Öffnung umfasst, wobei die Entfernung zwischen mindestens ±0,1 mm, genauer gesagt mindestens ±0,3 mm oder noch genauer gesagt mindestens ±0,5 mm schwanken kann.

3. Gehäuseanordnung nach Anspruch 1, **dadurch gekennzeichnet, dass** von einer der ersten Öffnung und zweiten Öffnung das Gas angepasst ist, um zu dem Raum hin zu strömen und die verbleibende erste Öffnung und zweite Öffnung zum Entfernen des Gases aus dem Raum dient.

4. Gehäuseanordnung nach Anspruch 1, **dadurch gekennzeichnet, dass** die erste Öffnung und zweite Öffnung in dieselbe Richtung öffnen.

5. Gehäuseanordnung nach Anspruch 1, **dadurch gekennzeichnet, dass** das Gehäuse zudem ein elastisches Element (45) zwischen der ersten Öffnung und der zweiten Öffnung umfasst.

6. Gehäuseanordnung nach Anspruch 5, **dadurch gekennzeichnet, dass** das elastische Element angepasst ist, um die zweite Wand mit der ersten Wand zu verbinden.

7. Gehäuseanordnung nach Anspruch 5 oder 6, **dadurch gekennzeichnet, dass** das elastische Element eine Zwischenwand zwischen der zweiten Wand und der ersten Wand ist.

8. Gehäuseanordnung nach Anspruch 1, **dadurch gekennzeichnet, dass** mindestens ein Teil des Kanals und des Körpers das elastische Element umfasst, das Biegen ermöglicht, das einen Unterschied zwischen gegenseitiger Lage der ersten Öffnung und der zweiten Öffnung ergibt.

9. Gehäuseanordnung nach Anspruch 1, **dadurch gekennzeichnet, dass** die erste Öffnung und zweite Öffnung parallel verlaufen.

10. Gehäuseanordnung nach Anspruch 1, **dadurch gekennzeichnet, dass** ein Fingerraum (51) zwischen dem Kanal und dem Körper bereitgestellt ist.

11. Anordnung zum Beatmen von Lungen eines Subjekts, umfassend:
ein Beatmungsgerät (3) zum Zuführen eines Atemgases für eine Einatmung und zum Empfangen eines Atemgases für eine Ausatmung;
einen Gasmischer (5) zum Zuführen eines frischen Gases für das atmende Subjekt; und
einen Atemkreis (7) zum Anschließen von Lungen (2) des Subjekts, des Beatmungsgeräts und des Gasmischers, wobei der Atemkreis einen Einatmungszweig (8) zum Bereitstellen eines Inspirationsgases umfasst, das das frische Gas für die Atmung des Subjekts umfasst; einen Ausatmungszweig (9) zum Abführen eines Expirationsgases; und eine Gehäuseanordnung (10) nach Anspruch 1.

12. Anordnung nach Anspruch 11, **dadurch gekennzeichnet, dass** die erste Öffnung und die zweite Öffnung, die angepasst sind, um in Strömungsverbindung mit dem Äußeren des Gehäuses zu sein, angepasst sind, um in Strömungsverbindung mit dem Atemkreis zu sein, und dass die erste Öffnung und zweite Öffnung in dieselbe Richtung öffnen.

13. Gehäuseanordnung nach Anspruch 11, **dadurch gekennzeichnet, dass** der Unterschied in Bezug auf gegenseitige Lage der ersten Öffnung und der zweiten Öffnung eine Änderung einer horizontalen Entfernung zwischen der ersten Öffnung und der zweiten Öffnung umfasst.

## Revendications

1. Ensemble de boîtier pour une substance (23) éliminant un composant de gaz respiratoire non souhaité d'un écoulement de gaz respiratoire, comprenant :
un corps (20) ayant un espace (22) pour recevoir ladite substance et ayant une première extrémité (27) et une seconde extrémité (28) qui est opposée à ladite première extrémité, en permettant l'écoulement du gaz respiratoire entre ces extrémités, une première paroi (25) entourant au moins une partie dudit espace et une première ouverture (38) en communication fluidique avec ladite première extrémité et avec l'extérieur dudit boîtier ;
un canal (21) en communication fluidique avec ladite seconde extrémité, lequel canal comprend un passage (35) entouré au moins en partie par une seconde paroi (36) pour guider l'écoulement de gaz et une seconde ouverture (39) en communication fluidique avec ledit passage et avec l'extérieur dudit boîtier ;
**caractérisé en ce que** ladite au moins une partie dudit canal est à même d'être séparée dudit corps pour permettre une variation en matière d'emplacement mutuel de ladite première ouverture et de ladite seconde ouverture et **en ce que** le canal est à même d'agir comme une poignée (50) pour le corps.

2. Ensemble de boîtier selon la revendication 1, **caractérisé en ce que** ladite variation quant à l'emplacement mutuel de ladite première ouverture et de ladite seconde ouverture comprend un changement de la distance entre ladite première ouverture et ladite seconde ouverture, laquelle distance peut varier d'au moins ± 0,1 mm, plus spécifiquement d'au moins ± 0,3 mm et de manière bien plus spécifique d'au moins ± 0,5 mm.

3. Ensemble de boîtier selon la revendication 1, **caractérisé en ce que**, depuis l'une de ladite première ouverture et de ladite seconde ouverture, le gaz est à même de s'écouler vers ledit espace et l'ouverture restante de ladite première ouverture et de ladite seconde ouverture est destinée à éliminer le gaz dudit espace.

4. Ensemble de boîtier selon la revendication 1, **caractérisé en ce que** ladite première ouverture et ladite seconde ouverture s'ouvrent dans la même direction.

5. Ensemble de boîtier selon la revendication 1, **caractérisé en ce que** ledit boîtier comprend également un élément élastique (45) entre ladite première ouverture et ladite seconde ouverture.

6. Ensemble de boîtier selon la revendication 5, **caractérisé en ce que** ledit élément élastique est à même de raccorder ladite seconde paroi à ladite première paroi.

7. Ensemble de boîtier selon la revendication 5 ou la revendication 6, **caractérisé en ce que** ledit élément élastique est une paroi intermédiaire entre ladite seconde paroi et ladite première paroi.

8. Ensemble de boîtier selon la revendication 1, **caractérisé en ce qu'**au moins une partie de l'un ou l'autre dudit canal et dudit corps comprend ledit élément élastique qui permet une flexion entraînant une variation entre les emplacements mutuels de ladite première ouverture et de ladite seconde ouverture.

9. Ensemble de boîtier selon la revendication 1, **caractérisé en ce que** ladite première ouverture et ladite seconde ouverture sont parallèles.

10. Ensemble de boîtier selon la revendication 1, **caractérisé en ce qu'**un espace d'un doigt (51) est ménagé entre ledit canal et ledit corps.

11. Aménagement destiné à ventiler les poumons d'un sujet, comprenant :
un ventilateur (3) pour fournir un gaz respiratoire pour une inspiration et recevoir un gaz respiratoire pour une expiration ;
un mélangeur de gaz (5) pour fournir un gaz frais pour la respiration du sujet ; et
un circuit respiratoire (7) pour raccorder les poumons (2) du sujet, ledit ventilateur et ledit mélangeur de gaz, ledit circuit respiratoire comprenant une branche d'inspiration (8) fournissant un gaz d'inspiration comprenant le gaz frais pour la respiration du sujet ; une branche d'expiration (9) pour décharger un gaz d'expiration ; et un ensemble de boîtier (10) selon la revendication 1.

12. Aménagement selon la revendication 11, **caractérisé en ce que** ladite première ouverture et ladite seconde ouverture, qui sont à même d'être en communication fluidique avec l'extérieur du boîtier, sont à même de se trouver en communication fluidique avec ledit circuit respiratoire et ladite première ouverture et ladite seconde ouverture s'ouvrent dans la même direction.

13. Ensemble de boîtier selon la revendication 11, **caractérisé en ce que** ladite variation quant à l'emplacement mutuel de ladite première ouverture et de ladite seconde ouverture comprend un changement de distance horizontale entre ladite première ouverture et ladite seconde ouverture.
